# EUROPEAN PATENT APPLICATION

(11) **EP 4 273 221 A1**
(43) Date of publication of application: **08.11.2023**
(21) Application number: 22216642.3
(22) Date of filing: 23.12.2022
(51) Int. Cl.: C12N 1/18, A23L 33/14, C12R 1/865

(54) **METHOD OF OBTAINING SACCHAROMYCES CEREVISIAE YEAST BIOMASS WITH INCREASED RESISTANCE TO OSMOTIC STRESS AND APPLICATION OF THE BIOMASS**

(30) Priority: 06.05.2022 PL 44111422
(71) Applicant: Uniwersytet Zielonogórski, 65-417 Zielona Góra (PL)
(72) Inventor: Kliks, Jaros aw, 66-120 Kargowa (PL); Ciepli ski, Mateusz, 66-003 Przytok (PL); Korycka-Korwek, Justyna, 65-128 Zielona Góra (PL); Kasprzak, Mariusz, 76-251 Kobylnica (PL); Mrówczy ska, Maria, 65-273 Zielona Góra (PL); Lachnicka, Dominika, 67-100 Nowa Sól (PL)
(74) Representative: JD&P Patent Attorneys Joanna Dargiewicz & Partners

(57) **Abstract**

The present invention relates to a method for obtaining *Saccharomyces cerevisiae* yeast biomass with increased resistance to osmotic stress, in which demineralized water in the amount of 88.21-97.69% of the total weight of the prepared biomass is added into the yeast dry mass in the amount of 0.98-2.65% of the total weight of the prepared biomass, demineralized water containing dissolved ions of calcium (144-180 mg/dm³), potassium (286-716 mg/dm³), magnesium (100-160 mg/dm³), ammonium nitrogen (106-360 mg/dm³), zinc (45-68 mg/dm³), and glucose (9.80-35.30 g/dm³), stirred for 5 hours at 130 rpm at 20°C, and then, after 5 hours, fatty acid ethyl esters in an amount of 0.1% to 5% per 1 dm³ of water are added to the mixture, and then the biomass is conditioned by mixing the components at 130 rpm at atmospheric pressure of 300 to 800 mbar with oxygen. The subject matter of the invention is also the use of *Saccharomyces cerevisiae* yeast biomass as a food additive, dietary supplement and precursor for obtaining fermentation biomass with improved fermentation parameters.

## Description

The present invention relates to a method of obtaining noble yeast *Saccharomyces cerevisiae* biomass with increased resistance to osmotic stress and use of the biomass in the production of food additives, dietary supplements, and fermentation biomass resistant to unfavorable osmotic conditions.

In the fermentation process, yeast utilizes simple sugars converting them into alcohol and energy under anaerobic conditions. As a result of these processes, the physicochemical conditions in the basic wort change as the concentration of simple sugars decreases, the content of ethanol and by-products of the fermentation process increases. The dynamics of these processes vary and depend mainly on the type of yeast and the initial sugar concentration. Each yeast strain has its own optimal conditions, outside of which the dynamics and efficiency of the process are reduced due to unfavorable osmotic pressure.

*Saccharomyces cerevisiae* yeast has been known since ancient times and is now widely used in the food industry, as well as for bioethanol production. This yeast is provided in several forms: compressed, dried, or freeze-dried. It can be used for production only after adding water, when the so-called "yeast milk" is formed and when the yeast cells regain their biological activity after water absorption. *Saccharomyces cerevisiae* is a candidate strain suitable for long-term large-scale microbial culture and is considered the most potential strain for large-scale production. *Saccharomyces cerevisiae* has the advantages of a short growth cycle, strong fermentation ability, and easy large-scale culture. It has always been the main object of basic and applied research and is widely used in food, medicine, and other fields. *Saccharomyces cerevisiae* is also used to ferment other industrially important metabolites.

In recent years, increasing attention has been paid to the selection of yeasts that show high resistance to unfavorable environmental conditions.

According to the publication by Monika CIOCH-SKONECZNY, EngD, Agnieszka PITEK, Eng., Pawe 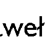 SATORA, EngD, UAK Prof., and Aneta PATER, M.Eng., entitled Rehydratacja drożdży piwowarskich (Rehydration of brewer's yeast) in Postqpy Techniki Przetwórstwa Spożywczego, 2018, vol. 2, pp. 79-83, http://yadda.icm.edu.pl/baztech/element/bwmeta1.element.baztech-d470b232-c3d7-48b7-a15b-29ba364cb0fb the purpose of rehydration is to restore, through contact with water, the properties of material subjected to drying—the properties which it had before drying. During the process, the tissues of the dried material absorb water, which leads to the material's weight and volume increase, and the leaching of substances, i.a. sugars, acids, minerals, and vitamins, from the rehydrated material. The loss of soluble components of the dry matter during rehydration depends primarily on the chemical composition and structure of the material. The rehydration process of yeast is crucial for it to regain its metabolic activity and to carry out fermentation. The survival rate of cells during drying depends on their prior condition. It is important to choose the right rehydration parameters, which largely depend on the type of strain used. Active dried yeast contains about 8% water. This is an insufficient amount for cells to regain their metabolic activity. Rehydration is therefore a necessary process before introducing them into the wort. There are many factors that influence the viability of yeast during this process. These include the intracellular concentration of trehalose, the length and temperature of rehydration, pH of the medium, the presence of nutrients, minerals, and the availability of ergosterol. The said factors affect the membrane structure by modifying permeability, causing changes in the flow of molecules and ions that determine the degree of viability of the rehydrated yeast cells. The rehydration temperature of active dried yeast ranges between 35 and 40°C.

In order for the cells to be rehydrated under safe conditions, the mineral content of the water in which the rehydration process is carried out has to be about 25 ppm. According to the law of osmosis, when the mineral concentration inside the yeast cell is higher than in the surrounding environment, the water will flow into the center of the cell, causing a rupture. For this reason, distilled water is not a good hydration medium for active dried yeast. Calcium ions and glucose can counteract the excessive efflux of intracellular substances from rehydrated cells. The former increase the rigidity of the membrane structure, affecting the repair of damaged cytoplasmic membranes, whereas the latter, glucose, penetrating into the cell, stimulates the formation of protein gels, the presence of which prevents the diffusion of intracellular substances.

In the dehydration (drying) process, water loss is an obvious and significant stress for yeast, and some studies identify it as a key factor responsible for reducing yeast viability. There are also many unfavorable factors, such as oxidative and osmotic stress. The efflux and influx of water from the cell during dehydration and rehydration can cause damage to the cell and destroy the very structure of the cell membrane. It is believed that shrinking of cells can cause their rupture during osmotic stress. In addition, it also leads to undesirable molecular interactions in the cell. There are mechanisms in yeast that increase its resistance to osmotic stress. The cell membrane contains membrane proteins, i.e. aquaporins, which under certain circumstances can facilitate osmotically driven water efflux, thus reducing the membrane damage. Under osmotic shock, the MAP-HOG kinase pathway is induced. Its aim is to accumulate glycerol in the cell, resulting in an equalization of osmolarity inside and outside the cell. This protects the cell from potential damage associated with increased osmotic pressure.

According to a publication by W 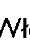 odzimierz Grajek and Daria Szymanowska, entitled "Stresy środowiskowe dzia 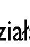 aj 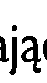 ce na drożdże *Saccharomyces cerevisiae* w procesie fermentacji etanolowej" (The influence of environmental stresses on *Saccharomyces cerevisiae* yeast in ethanol fermentation) in: Review papers, Poznań University of Life Sciences, 2008, pp. 46-63, one of the main yeast metabolites accumulated in the cell in stress conditions is trehalose, and the role of this sugar is to protect the stability of hydrated compounds and to seal membranes, preventing the loss of electrolytes and soluble cellular components. In addition, in the fermentation process for alcohol production, two-step starch hydrolysis or the process of simultaneous starch hydrolysis and ethanol fermentation is used to reduce osmotic stress. Attempts are made to reduce the osmotic pressure caused by ethanol by continuously removing ethanol from the fermenting mash. Cells limit the effects of osmotic stress by reducing cell volume, which has a negative effect on their viability. Under osmotic stress, in addition to cell surface area reduction, damage to the cytoplasmic membrane can occur. This is especially true for changes in membrane proteins, which under severe dehydration caused by the water binding by an extracellular osmogenic substance undergo denaturation or aggregation. These changes can be observed using Fourier transform infrared spectroscopy. Osmogenic substances, especially sugars and alcohols, have a protective effect, stabilizing protein molecules. Another effect of changes in osmotic pressure is the lateral diffusion of fatty acids in the phospholipid bilayer.

In order to reduce the osmotic stress, genetically modified strains of *Saccharomyces cerevisiae* yeast with high resistance to osmotic stress are produced.

From the CN112941119 patent application, a method is known for obtaining a modified *Saccharomyces cerevisiae* strain producing fatty acid ethyl ester, a method which improves the production of fatty acid ethyl esters in *Saccharomyces cerevisiae* yeast, including without limitation a step of fermentation and culturing of *Saccharomyces cerevisiae* for 30-40 hours, during which 1-3% rapeseed oil and ethanol are added at a flow rate of 6-10 mL/h; when the glucose concentration is lower than 4-6 g/L, glucose supplementation is initiated, and the glucose concentration in the fermentor is maintained at 5-10 g/L. After fermentation, a fermentation broth containing fatty acid ethyl ester is obtained. During the fermentation and culturing process, the pH is maintained at 5-6, the speed of rotation is 180-220 rpm for 30-40 hours before fermentation, the airflow is 2-3 vvm, the speed of rotation after 30-40 hours is 350-450 rpm, and the airflow is 4-6 wm. The following are used as the fermentation medium: glucose 50-70, YNB 6-7, yeast powder 15-20, peptone 2-4, K2HPO47-8, KH2PO49-10, acetic acid 2-4.

The object of the invention disclosed in CN1944657A patent application is to solve the problem of alcohol tolerance by *Saccharomyces cerevisiae* in the process of alcohol thick mash fermentation, and to reduce the toxic effects of highly concentrated alcohol on *Saccharomyces cerevisiae,* thus providing a way to improve the tolerance of *Saccharomyces cerevisiae* to alcohol. The presence of fatty acids, especially long-chain fatty acids, in the cell membrane can weaken the effect of ethanol on cell membrane permeability and reduce the efflux of intracellular lysates. The tolerance of bacteria to alcohol increases with the increase of unsaturation of cell membranes with fatty acids. The invention uses soybean powder as a fermentation promoter in alcoholic fermentation. Soybean, rich in protein and fatty acids, can make saccharomyces resist the increased membrane fluidity caused by alcohol and maintain cell membrane stability.

The JPS6460370A patent application discloses a method of *Saccharomyces cerevisiae* yeast cultivating, in which a lipid-a fatty acid with 10-26 carbon atoms or a salt thereof, preferably myristic acid, palmitic acid, palmitoleic acid, oleic acid, t-vaccenic acid, linoleic acid and arachidonic acid and/or an osmotic pressure regulator, such as sorbitol, is added to the medium of the modified strain. Preferably, the lipid is combined with an osmotic pressure regulator. The fatty acid is added to the medium at a concentration of about 0.005-0.4% (w/v), preferably 0.01-0.2%. When it is added at a concentration of less than 0.005%, the desired effects cannot be obtained, and when it is added at a concentration of more than 0.4%, production may decrease. Although fatty acid is generally added to the medium in a sufficient amount before incubation, it can be added at the initial step of the incubation.

From the PL218665 patent it is known a method of obtaining yeast milk based on yeast, water, and nutrients, characterized in that water at a temperature of 20-35°C in an amount of 50-70% of the total weight of the yeast milk to be prepared is added into a tank and yeast is thrown in an amount of 30-50% of the total weight, after which the mixing process is started for 1 to 2.5 hours, and then at 3 to 5 hours the nutrients are added portionwise in the amount of 0-15%, and oxygen is supplied to the tank through aeration tubes.

Currently, there are no methods, other than through modification of the *S. cerevisiae* yeast strain, to produce biomass that is characterized by significant accumulation of the polyunsaturated fatty acids: C18:2 n-6 linoleic acid and C18:3 n-3 linolenic acid (ALA) in the *S. cerevisiae* yeast cell wall and resistance to osmotic stress.

The object of the invention is to solve the technical problem related to the *S. cerevisiae* yeast biomass by increasing the resistance of S. *cerevisiae* yeast cells to the osmotic stress prevailing in the fermentation medium and increasing the accumulation of the polyunsaturated fatty acids: C18:2 n-6 linoleic acid and C18:3 n-3 linolenic acid (ALA), in the cell wall of *S. cerevisiae* yeast.

Thus, the subject matter of the present invention is a method of obtaining *Saccharomyces cerevisiae* yeast biomass with increased resistance to osmotic stress, wherein the rehydration process of *Saccharomyces cerevisiae* yeast dry mass in water at 20°C with the addition of nutrients and supply of oxygen is characterized according to the invention in that to the yeast dry mass in the amount of 0.98-2.65% of the total weight of the prepared biomass demineralized water is added in the amount of 88.21-97.69% of the total weight of prepared biomass, containing dissolved ions of calcium (144-180 mg/dm³), potassium (286-716 mg/dm³), magnesium (100-160 mg/dm³), ammonia nitrogen (106-360 mg/dm³), zinc (45-68 mg/dm³), and glucose (9.80-35.30 g/dm³), is stirred for 5 hours at 130 rpm at 20°C, and then after 5 hours fatty acid ethyl esters are added to the mixture in an amount of 0.1% to 5% per 1 dm³ of water, and then the biomass is conditioned by mixing the ingredients at 130 rpm at an atmospheric pressure of 300 to 800 mbar with oxygen.

Preferably, the *Saccharomyces cerevisiae* SafSpirit M-1 yeast is used.

Preferably, ethyl esters of linseed oil fatty acids are used.

Preferably, the biomass is centrifuged, then frozen at -40°C to 75°C and freeze-dried for 24 hrs at 20°C and 0.8-0.08 mbar.

Preferably, the biomass is centrifuged, then frozen at -40°C to 75°C and freeze-dried for 24 hrs at 35°C and 0.8-0.08 mbar.

Preferably, the biomass of *Saccharomyces cerevisiae* yeast is obtained either in form of yeast milk or in dry form.

The subject matter of the invention is also the use of the *Saccharomyces cerevisiae* yeast biomass obtained by the above-described method as a food additive.

The subject matter of the invention is also the use of the *Saccharomyces cerevisiae* yeast biomass obtained by the above-described method as a dietary supplement.

The subject matter of the invention is also the use of the *Saccharomyces cerevisiae* yeast biomass obtained by the above-described method as a precursor for obtaining fermentation biomass with improved fermentation parameters.

The method according to the invention allows a significant accumulation of C18:2 n-6 and C18:3 n-3 fatty acids (ALA) in yeast cells, such that:
- The biomass obtained in form of yeast milk can be used as a precursor for the production of fermentation biomass resistant to unfavorable osmotic conditions, as a food additive-including a bakery additive-and as a dietary supplement;
- The biomass obtained after the freeze-drying process, i.e., in dry form, can act both as a foodstuff characterized by a high content of easily digestible polyunsaturated fatty acids or as a dietary supplement, and as an osmoactive biomass, for the fermentation worts with a high content of sugars.

Thanks to the method according to the invention allowing the conditioning of *S. cerevisiae* yeast in a medium rich in ethyl esters of linseed oil fatty acids and simple sugars, a biomass is created that accumulates C18:2 n-6 and C18:3 n-3 unsaturated fatty acids (ALA) in its cell walls. This accumulation has a positive effect on cell turgor preservation-the cells do not dehydrate under severe osmotic stress, and on increasing the dynamics of the process-the amount of ethanol (grams) obtained from 100 mL of the medium during 1 hour.

The invention is presented in more detail in embodiments:

### Example 1a

Table 1 shows the composition of *Saccharomyces cerevisiae* yeast biomass in the form of yeast milk, in which the components were combined using a minimum dosage of individual bioactive substances. The components of the yeast milk, with appropriate processing conditions, make it possible to obtain biomass with high tolerance to osmotic stress. Depending on the purpose of biomass production, different dosages of ethyl esters of linseed oil fatty acids can be used.

**Table 1. Composition of Saccharomyces cerevisiae yeast biomass in the form of yeast milk with minimal doses of components**

| **Component** | **Mass [g]** | **Percentage** |
|---|---|---|
| *Saccharomyces cerevisiae* yeast | 10.0 | 0.98 |
| Demineralized water | 1000.0 | 97.69 |
| Calcium chloride | 0.4 | 0.04 |
| Potassium hydrogen phosphate KH₂PO₄ | 1.0 | 0.10 |
| Ammonium sulfate (NH₄)₂SO₄ | 0.5 | 0.05 |
| Zinc sulfate heptahydrate 7 H₂O x ZnSO₄ | 0.2 | 0.02 |
| Magnesium sulfate MgSO₄ | 0.5 | 0.05 |
| Glucose | 10.0 | 0.98 |
| Ethyl esters of linseed oil fatty acids | 1.0 | 0.10 |
| Total | 1023.6 | 100.00 |

*Saccharomyces cerevisiae* SafSpirit M-1 noble yeast is prepared by weighing 10 g of dry mass. The yeast dry mass is placed in a stirred (130 rpm) bioreactor, 1 dm³ of demineralized water at 20°C is added, containing dissolved ions of calcium (144 mg/dm³), potassium (286 mg/dm³), magnesium (100 mg/dm³), ammonia nitrogen (106 mg/dm³), zinc (45 mg/dm³), and glucose (9.80 g/dm³).

The yeast rehydration process is carried out for 5 hours, maintaining a constant bioreactor mantle temperature of 20°C. After the indicated time, ethyl esters of linseed oil fatty acids are added to the mixture in the amount of 1 g of esters per 1 dm³ of demineralized water resulting in concentration of 0.1%.

The process is then carried out according to the guidelines in Table 2. At all times the mixing is carried out at 130 rpm.

**Table 2. Guidelines for the yeast biomass conditioning process**

| **Duration of the process** | **Vacuum** | **Aeration** |
|---|---|---|
| **2 h** | Atmospheric pressure | 2 dm³/min |
| **2 h** | 300 mbar vacuum | 0 dm³/min |
| **2 h** | Atmospheric pressure | 2 dm³/min |
| **2 h** | 300 mbar vacuum | 0 dm³/min |

Biomass aeration is carried out through the appropriate port, with air fed from a pump or compressed air cylinder while maintaining proper purity-0.14 µm filter is mounted immediately before the bioreactor air inlet.

### Example 1b

The *Saccharomyces cerevisiae* yeast biomass obtained in Example 1a in the form of yeast milk is used directly or processed to obtain dry yeast.

The yeast milk undergoes separation in a separator. The non-polar phase accumulates in the upper layer, while the aqueous phase containing yeast cells accumulates in the lower layer in the separator. After the phase separation, the liquid is immediately subjected to separation in a bactofuge.

A stream of yeast milk is fed at a volumetric rate of 30 mL/min into a bactofuge operating at 5000 rpm. The resulting centrifuged biomass is spread on trays and shock-frozen at - 40°C for 24 hrs.

After this time, the trays are placed in the freeze-dryer chamber. Freeze-drying is carried out at a tray temperature of 20°C, a pressure of 0.8 mbar, for 24 hrs.

In the embodiment variant, the centrifuged biomass is spread on trays and shock-frozen at -75°C for 24 hrs, after which freeze-drying is carried out at a tray temperature of 20°C, a pressure of 0.08 mbar, for 24 hrs.

The powder obtained after freeze-drying is vacuum-packed under a vacuum of 30 mbar.

### Example 2a

Table 3 shows the composition of *Saccharomyces cerevisiae* yeast biomass in the form of yeast milk, where the components were combined using maximal doses of each substance. The components of the yeast milk, with appropriate processing conditions, make it possible to obtain biomass with high tolerance to osmotic stress. Depending on the purpose of biomass production, different dosages of ethyl esters of linseed oil fatty acids can be used.

**Table 3. Composition of Saccharomyces cerevisiae yeast biomass in the form of yeast milk with maximal doses of components**

| **Component** | **Mass [g]** | **Percentage** |
|---|---|---|
| *Saccharomyces cerevisiae* yeast | 30.0 | 2.65 |
| Demineralized water | 1000.0 | 88.21 |
| Calcium chloride | 0.6 | 0.05 |
| Potassium hydrogen phosphate KH₂PO₄ | 3.0 | 0.26 |
| Ammonium sulfate (NH₄)₂SO₄ | 2.0 | 0.18 |
| Zinc sulfate heptahydrate 7 H₂O x ZnSO₄ | 0.4 | 0.04 |
| Magnesium sulfate MgSO₄ | 0.9 | 0.08 |
| Glucose | 40.0 | 3.53 |
| Ethyl esters of linseed oil fatty acids | 56.7 | 5.00 |
| Total | 1176.9 | 100.00 |

*Saccharomyces cerevisiae* SafSpirit M-1 noble yeast is prepared by weighing 30 g of dry biomass. The yeast dry mass is placed in a stirred (130 rpm) bioreactor, 1 dm³ of demineralized water at 20°C is added, containing dissolved ions of: calcium (180 mg/dm³), potassium (716 mg/dm³), magnesium (160 mg/dm³), ammonia nitrogen (360 mg/dm³), zinc (68 mg/dm³), and glucose (35.30 g/dm³).

The yeast rehydration process is carried out for 5 hours, maintaining a constant bioreactor mantle temperature of 20°C. After the indicated time, ethyl esters of linseed oil fatty acids are added to the mixture in the amount of 56.7 g of esters per 1 dm³ of demineralized water, which is a concentration of 5.0%.

The process is then carried out according to the guidelines in Table 4. At all times the mixing is carried out at 130 rpm.

**Table 4. Guidelines for the yeast biomass conditioning process**

| **Duration of the process** | **Vacuum** | **Aeration** |
|---|---|---|
| **2 h** | Atmospheric pressure | 6 dm³/min |
| **2 h** | 800 mbar vacuum | 0 dm³/min |
| **2 h** | Atmospheric pressure | 6 dm³/min |
| **2 h** | 800 mbar vacuum | 0 dm³/min |

Biomass aeration is carried out through the appropriate port, with air fed from a pump or compressed air cylinder while maintaining proper purity-0.14 µm filter is mounted immediately before the bioreactor air inlet.

### Example 2b

The *Saccharomyces cerevisiae* yeast biomass obtained in Example 2a in the form of yeast milk is used directly or processed to obtain dry yeast.

The yeast milk undergoes separation in a separator. The non-polar phase accumulates in the upper layer, while the aqueous phase containing yeast cells accumulates in the lower layer in the separator. After the phase separation, the liquid is immediately subjected to separation in a bactofuge.

A stream of yeast milk is fed at a volumetric rate of 300 mL/min into a bactofuge operating at 12,000 rpm. The resulting centrifuged biomass is spread on trays and shock-frozen at -40°C for 24 hrs.

After this time, the trays are placed in the freeze-dryer chamber. Freeze-drying is carried out at a tray temperature of 35°C, a pressure of 0.08 mbar, for 24 hrs.

In the embodiment variant, the centrifuged biomass is spread on trays and shock-frozen at -75°C for 24 hrs, after which freeze-drying is carried out at a tray temperature of 35°C, a pressure of 0.03 mbar, for 24 hrs.

The powder obtained after freeze-drying is vacuum-packed under a vacuum of 30 mbar.

In the compositions indicated in Examples 1a, 1b, 2a, 2b, *S.* cerevisiae SafSpirit M-1 was used, however, since *S. cerevisiae* yeast has similar characteristics and similar structure, but differs in specific species characteristics, the invention can be applied to *S. cerevisiae* yeast other than SafSpirit M-1. The C18:2 n-6 and C18:3 n-3 acid (ALA) absorption will be the same characteristic of all *S. cerevisiae.*

### Example 3 - Use of Saccharomyces cerevisiae yeast biomass as a dietary supplement

*Saccharomyces cerevisiae* yeast biomass was produced in the form of yeast milk according to the procedure described in Example 1a or 2a, using different dosage levels of ethyl esters of linseed oil fatty acids, designated BFO - no addition of esters to the yeast milk composition, BF1.25 - 1.25% addition of esters, BF2.50 - 2.50% addition of esters, BF3.75-3.75% addition of esters, and BF5.00 - 5.00% addition of esters. Significant differences were observed between the content of C18:2 n-6 and C18:3 n-3 acids (ALA) determined in yeast cells depending on the dosage level of ethyl esters of linseed oil fatty acids to the prepared fermentation milk. The resulting biomass after drying was a rich source of polyunsaturated acids.

Based on the data in Table 5, it can be observed that as the percentage of ethyl esters of linseed oil fatty acids increased, the level of C18:2 n-6 and C18:3 n-3 (ALA) acids, which are a fraction of polyunsaturated essential fatty acids, increased significantly in the studied biomass. These acids play an important role in the proper functioning of the human body. The use of biomass according to the invention as a dietary supplement containing a high percentage of these fatty acids in bound form in biomass can result in their significantly better absorption in the gastrointestinal tract.

**Table 5. Fatty acid profile of the centrifuged yeast cells**

| | **BF0 [%]** | **BF1.25 [%]** | **BF2.50 [%]** | **BF3.75 [%]** | **BF5.00 [%]** |
|---|---|---|---|---|---|
| **C10:0** | 4.24 | 2.25 | 0.39 | 0.43 | 0.43 |
| **C16:0** | 24.64 | 18.69 | 22.14 | 18.78 | 14.97 |
| **C16:1** | 26.67 | 9.61 | 7.11 | 6.88 | 5.81 |
| **C18:0** | 16.12 | 11.59 | 15.25 | 10.44 | 8.10 |
| **C18:1** | 28.33 | 22.90 | 21.92 | 20.63 | 18.72 |
| **C18:2 n-6** | 0.01 | 6.11 | 5.29 | 8.38 | 9.62 |
| **C18:3 n-3 (ALA)** | 0.01 | 28.84 | 27.91 | 34.46 | 42.35 |

### Example 4 - Use of Saccharomyces cerevisiae yeast biomass as a food additive

Prepared *Saccharomyces cerevisiae* yeast biomass in dry form with a high percentage of C18:2 n-6 and C18:3 n-3 (ALA) acids can be a good food additive both in active form, i.e. in products, such as bakery products, in which yeast activity does not impair the sensory characteristics or shelf life, and in inactive biomass form, i.e. biomass in dry form.

The inactive biomass form may have a much wider use due to its lack of negative effect on the product sensory characteristics and the occurrence of possible fermentation, which could reduce the shelf life. The inactive form is a biological carrier of C18:2 n-6 and C18:3 n-3 acids allowing to increase the nutritional value of the product while lacking the unpleasant aftertaste of rancidity associated with the presence of free fatty acids. Inactive yeast enriched in C18:2 n-6 and C18:3 n-3 acids can be preferably used as an additive to:
- Fruit yogurts (0.1-1%);
- Ripened cheeses (0.5-1%);
- Wheat and wheat-rye bread (1-2%);
- Rye bread (0.5-1%);
- Pâtés (0.5-2%);
- Cured meats (0.1-3%);
- Processed fruit and fruit and vegetable products (0.1-1%).

### Example 5 - Use of Saccharomyces cerevisiae yeast biomass as a precursor to obtain fermentation biomass with improved fermentation parameters

Prepared *Saccharomyces cerevisiae* yeast biomass in the form of yeast milk containing a high percentage of C18:2 n-6 and C18:3 n-3 (ALA) acids can be a precursor for obtaining fermentation biomass with improved fermentation parameters.

Batch fermentations were prepared as follows:
10 mL inoculums in the form of *Saccharomyces cerevisiae* yeast biomass obtained in Example 2 incubated for 24 hrs at 20°C were transferred quantitatively to 300 mL flasks. Then, to each flask batch fermentation (BF) sample of ethyl esters of linseed oil fatty acids was added in amounts of BFO (0.00%), BF1.25 (1.25%), BF2.50 (2.50%), BF3.75 (3.75%) and BF5.00 (5.00%).

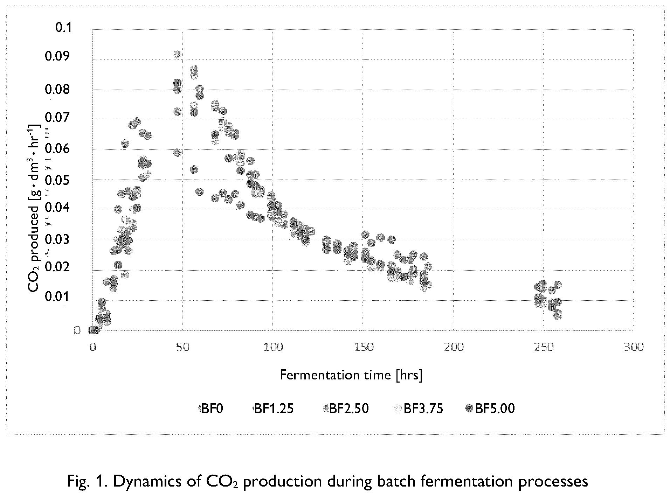

Fig. 1 shows the batch process fermentation dynamics. A much lower process dynamics can be observed for the BFO batch, which was prepared with yeast inoculums not supplemented with fatty acid esters. For ester-supplemented batches, much higher fermentation dynamics were recorded, with the maximum recorded at the 47th hour of the process for the BF3.75 batch.

Table 6 presents the effect of supplementation with fatty acid ethyl esters on improving the accumulation of the polyunsaturated fatty acids: C18:2 n-6 linoleic acid and C18:3 n-3 linolenic acid (ALA) in the cell wall of *S. cerevisiae* yeast and improving resistance to osmotic stress. A significantly higher concentration of ethanol (11.39%) was determined in the BF5.00 batch containing 5% ester addition to biomass than in BFO (10.39%), the cells with supplementation accumulated glycerol as an osmoprotective agent to a significantly greater extent. Also, the diameter of yeast cells in the supplemented batches differed significantly from the values obtained in the blank sample.

**Table 6. Fermentation effects for the batches supplemented with ethyl esters of linseed oil fatty acids**

| | **BF0** | **BF1.25** | **BF2.50** | **BF3.75** | **BF5.00** |
|---|---|---|---|---|---|
| **Ethanol [% v/v]** | 10.39 ± 0.20 | 10.97 ± 0.24 | 11.17 ± 0.31 | 11.21 ± 0.14 | 11.39 ± 0.18 |
| **Glycerol [g·dm⁻³**] | 8.11 ± 0.07 | 8.40 ± 0.10 | 8.69 ± 0.09 | 8.66 ± 0.14 | 9.03 ± 0.11 |
| **Cell size [µm]** | 5.17 ± 0.11 | 5.30 ± 0.06 | 5.40 ± 0.16 | 5.37 ± 0.21 | 5.80 ± 0.24 |

## Claims

1. A method of obtaining *Saccharomyces cerevisiae* yeast biomass with increased resistance to osmotic stress, in which the rehydration process of *Saccharomyces cerevisiae* yeast dry mass is carried out in water at 20°C with the addition of nutrients and supply of oxygen, **characterized in that** demineralized water in the amount of 88.21-97.69% of the total weight of the prepared biomass is added into the yeast dry mass in the amount of 0.98-2.65% of the total weight of the prepared biomass, demineralized water containing dissolved ions of calcium (144-180 mg/dm³), potassium (286-716 mg/dm³), magnesium (100-160 mg/dm³), ammonia nitrogen (106-360 mg/dm³), zinc (45-68 mg/dm³), and glucose (9.80-35.30 g/dm³), stirred for 5 hours at 130 rpm at 20°C, and then after 5 hours fatty acid ethyl esters in an amount of 0.1% to 5% per 1 dm³ of water are added to the mixture, and then the biomass is conditioned by mixing the components at 130 rpm at an atmospheric pressure of 300 to 800 mbar with oxygen.

2. The method according to claim 1 **characterized in that** *Saccharomyces cerevisiae* SafSpirit M-1 yeast is used.

3. The method according to claim 1 or 2, **characterized in that** ethyl esters of linseed oil fatty acids are used.

4. The method according to any of the claims 1 to 3, **characterized in that** the biomass is centrifuged, then frozen at -40°C to -75°C and freeze-dried for 24 hrs at 20°C and at a pressure of 0.8-0.08 mbar.

5. The method according to any of the claims 1 to 3, **characterized in that** the biomass is centrifuged, then frozen at -40°C to -75°C and freeze-dried for 24 hrs at 35°C and at a pressure of 0.08-0.03 mbar.

6. The method according to any of the claims 1 to 3, **characterized in that** the *Saccharomyces cerevisiae* yeast biomass is obtained in the form of yeast milk.

7. The method according to any of the claims 1 and 4 and 5, **characterized in that** a dry *Saccharomyces cerevisiae* yeast biomass is obtained.

8. The use of *Saccharomyces cerevisiae* yeast biomass obtained by the method defined in any of the claims 1-7 as a food additive.

9. The use of *Saccharomyces cerevisiae* yeast biomass obtained by the method defined in any of the claims 1-7 as a dietary supplement.

10. The use of *Saccharomyces cerevisiae* yeast biomass obtained by the method defined in any of the claims 1-7 as a precursor for obtaining fermentation biomass with improved fermentation parameters.
